(19) Europäisches Patentamt
European Patent Office
Office européen des brevets

(11) Veröffentlichungsnummer: **0 322 359 B1**

(12) **EUROPÄISCHE PATENTSCHRIFT**

(45) Veröffentlichungstag der Patentschrift: **16.06.93**

(51) Int. Cl.[5]: **C09B 57/04**, C08K 5/34, //C07D487/04,(C07D487/04, 249:00,237:00)

(21) Anmeldenummer: **88810858.6**

(22) Anmeldetag: **13.12.88**

# (54) Neue Isoindolinpigmente enthaltend mindestens einen Triazolopyrimidonrest.

(30) Priorität: **21.12.87 CH 4986/87**

(43) Veröffentlichungstag der Anmeldung: **28.06.89 Patentblatt 89/26**

(45) Bekanntmachung des Hinweises auf die Patenterteilung: **16.06.93 Patentblatt 93/24**

(84) Benannte Vertragsstaaten: **CH DE FR GB IT LI**

(56) Entgegenhaltungen:
**EP-A- 0 088 920**
**EP-A- 0 185 618**
**EP-A- 0 208 346**
**EP-A- 0 220 458**
**DE-A- 1 597 615**

(73) Patentinhaber: **CIBA-GEIGY AG**
**Klybeckstrasse 141**
**CH-4002 Basel(CH)**

(72) Erfinder: **Jost, Max**
**Rebgartenweg 20**
**CH-4104 Oberwil(CH)**
Erfinder: **Burdeska, Kurt, Dr.**
**Laufenburgerstrasse 30**
**CH-4058 Basel(CH)**
Erfinder: **von der Crone, Jost, Dr.**
**La Dey**
**CH-1732 Arconciel(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Isoindoline enthaltend mindestens einen s-Triazolo[2,3-a]-pyrimidin-5,7-dion-rest, ein Verfahren zu deren Herstellung sowie ihre Verwendung als Pigmente zum Färben von hochmolekularem organischem Material.

Isoindolinpigmente sind seit langer Zeit dem Fachmann bekannt. So sind Isoindoline enthaltend einen Barbitursäure- oder einen Benzimidazolo-pyrimidin-dion-rest beispielsweise im US-Patent Nr. 4 262 120 bzw. im US-Patent Nr. 4 525 591 beschrieben. Obwohl sich diese Isoindoline allgemein durch gute Pigmenteigenschaften in der Applikation auszeichnen, vermögen sie den heutigen Anforderungen der Praxis nicht immer zu genügen.

Es ist nun gefunden worden, dass sich Isoindoline enthaltend mindestens einen s-Triazolo[2,3-a]-pyrimidin-5,6-dion-rest durch hervorragende Pigmenteigenschaften auszeichnen.

Die Erfindung betrifft demnach Isoindoline der Formel I

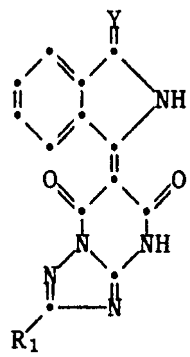
(I),

worin

Y für einen Rest der Formeln II, III, IV, IVa, V oder VI

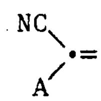
,
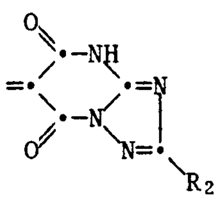
,
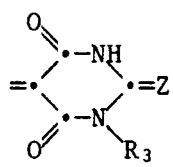
,
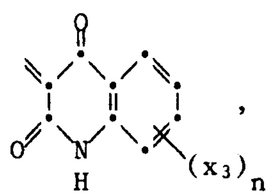
,

(II) (III) (IV) (IVa)

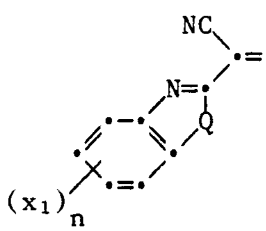
,
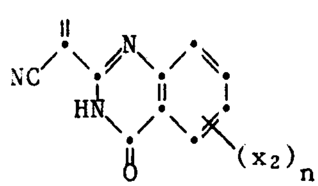
steht, wobei

(V) (VI)

$R_1$ und $R_2$ unabhängig voneinander -H, $-CONH_2$, $-COOC_1$-$C_4$-Alkyl, $C_1$-$C_3$-Alkyl, unsubstituiertes oder durch ein bis drei gleiche oder verschiedene Substituenten bestehend aus den Gruppen Halogen, $C_1$-$C_3$-Alkyl oder $C_1$-$C_3$-Alkoxy substituiertes Phenyl bedeuten, $R_3$ -H, $C_1$-$C_4$-Alkyl oder unsubstituiertes oder durch ein bis zwei gleiche oder verschiedene Substituenten bestehend aus den Gruppen Halogen, $C_1$-$C_3$-Alkyl oder $C_1$-$C_3$-Alkoxy substituiertes Phenyl ist, Z und Q für O, S oder NH stehen, und A eine Gruppe der Formeln -CN, -COO-$C_1$-$C_4$-Alkyl oder $-CONHR_4$ ist, wobei $R_4$ -H, $C_1$-$C_4$-Alkyl, Benzyl, unsubstituiertes oder ein- oder mehrfach gleich oder verschieden durch Halogen, $C_1$-$C_4$-Alkyl, $C_1$-$C_4$-Alkoxy, eine Gruppe $-COR_5$, $-NHCOC_1$-$C_4$-Alkyl und

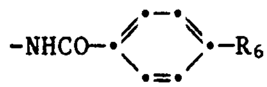

substituiertes Phenyl ist,

worin $R_5$ $C_1$-$C_4$-Alkoxy, -$NH_2$ oder -$NHC_1$-$C_4$-Alkyl, und $R_6$ Wasserstoff, Halogen oder $C_1$-$C_4$-Alkyl bedeuten, ferner $R_4$ unsubstituiertes oder durch Halogen, Methyl oder Methoxy mono- oder disubstituiertes Naphthyl ist, und $x_1$, $x_2$ und $x_3$ unabhängig voneinander Halogen, Methyl, Methoxy oder -$NHCOCH_3$ und n die Zahl 0,1 oder 2 darstellen.

Bedeuten etwaige Substituenten $C_1$-$C_3$-Alkyl oder $C_1$-$C_4$-Alkyl, so handelt es sich z.B. um Methyl, Ethyl, n-Propyl, Isopropyl, ferner n-Butyl, sec.-Butyl oder tert.-Butyl.

Bedeuten etwaige Substituenten $C_1$-$C_3$-Alkoxy oder $C_1$-$C_4$-Alkoxy, so handelt es sich z.B. um Methoxy, Ethoxy, n-Propoxy, Isopropoxy, ferner n-Butoxy, sec.-Butoxy oder tert.-Butoxy.

Bedeuten etwaige Substituenten Halogen, so handelt es sich z.B. um Jod, Brom, Fluor und insbesondere Chlor.

Bedeutet $R_4$ einen Naphthylrest, so handelt es sich um einen $\beta$- und insbesonders um einen $\alpha$-Naphthylrest.

Von besonderem Interesse sind Isoindoline der Formel I, worin Y für einen Rest der Formel

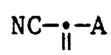

steht, und $R_1$ unsubstituiertes oder durch ein bis drei gleiche oder verschiedene Substituenten bestehend aus den Gruppen Chlor, Methyl oder Methoxy substituiertes Phenyl bedeutet, und A eine Gruppe der Formel -$CONHR_4$ ist, wobei $R_4$ unsubstituiertes oder ein- oder zweifach gleich oder verschieden durch -Cl, -$CH_3$, -$OCH_3$, -$COOCH_3$, -$COOC_2H_5$, -$CONH_2$, -$NHCOCH_3$ und -$NHCOC_6H_5$ substituiertes Phenyl bedeutet.

Von ganz besonderem Interesse sind Isoindoline der Formel I, worin $R_1$ Phenyl und Y eine Gruppe der Formel

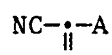

bedeuten, wobei A unsubstituiertes oder durch 1 oder 2 Chloratome substituiertes Phenyl ist.

Zu den erfindungsgemässen Isoindolinen gelangt man, wenn man z.B. das Diiminoisoindolin der Formel

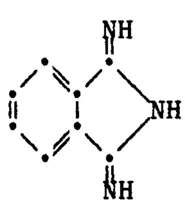

in beliebiger Reihenfolge mit je einem Mol einer Verbindung der Formel VII

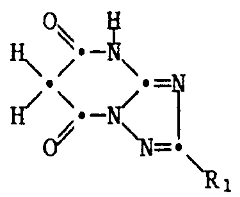

(VII)

und einer den Formeln II, V und VI entsprechenden cyanmethylenaktiven Verbindung oder einer den Formeln III, IV und IVa entsprechenden Diketomethylen-Verbindung, worin A, $R_1$, $R_2$, $R_3$, Z, Q, $x_1$, $x_2$, $x_3$ und n die oben angegebene Bedeutung haben, in Analogie zu an sich bekannten Verfahren, wie sie z.B. in den US-Patenten Nr. 4 262 120 und Nr. 4 426 533 beschrieben sind, kondensiert.

Zweckmässig aber können durch Umsetzung von 1,3-Diiminoisoindolin mit 1 Mol einer Verbindung der Formel VII oder 1 Mol einer der Formeln II, III, IV, IVa, V oder VI entsprechenden cyanmethylenaktiven oder Diketomethylen-Verbindung zuerst Monokondensationsprodukte der Formeln

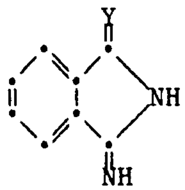

oder

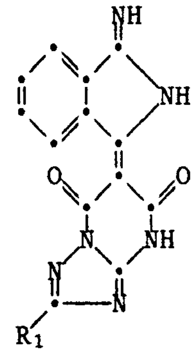

gebildet werden, welche dann durch Reaktion mit einer den Formeln II bis VI entsprechenden cyanmethylenaktiven oder Diketomethylen-Verbindung zu den Biskondensationsprodukten der Formel I umgesetzt werden. Y und $R_1$ haben die oben angegebene Bedeutung.

Die Kondensation des Diiminoisoindolins mit den cyanmethylenaktiven Verbindungen gemäss Formeln II, V und VI zu den Monokondensationsprodukten erfolgt vorzugsweise in einem organischen Lösungsmittel, beispielsweise einem aliphatischen Alkohol mit 1 bis 4 C-Atomen, wie Methanol, Ethanol, Isopropanol und Butanol, ferner Glykolen oder Glykolethern, offenkettigen oder cyclischen Amiden, wie Dimethylformamid, Dimethylacetamid oder N-Methylpyrrolidon, oder in Mischungen der vorgenannten Lösungsmittel sowie in Mischungen der genannten Lösungsmittel mit Wasser. Ein geringer Ueberschuss an Diiminoisoindolin kann vorteilhaft sein. Die Menge an Lösungsmittel ist an sich unkritisch und wird durch die Rührbarkeit bzw. Mischbarkeit des Reaktionsansatzes bestimmt. Die Umsetzung erfolgt in der Regel bei Temperaturen unterhalb von 100 °C.

Die Mono- oder Biskondensation des Diiminoisoindolins oder die Kondensation der obigen Monokondensationsprodukte mit den Diketomethylen-Verbindungen gemäss Formeln III, IV und IVa erfolgt zweckmässig in aliphatischen Mono- oder Dicarbonsäuren, insbesondere in aliphatischen Monocarbonsäuren, wie Essigsäure oder Propionsäure, bei Temperaturen zwischen 50 und 150 °C.

Durch geeignete Reaktionsführung ist es möglich, auch beide Reaktionsschritte ohne Zwischenisolierung der Monokondensationsprodukte im gleichen Reaktionsgefäss durchzuführen.

Die der Formeln II, IV, IVa, V und VI entsprechenden Cyanmethylen- oder Diketomethylen-Verbindungen stellen bekannte Verbindungen dar.

Dagegen sind die Diketomethylen-Verbindungen der Formel VII (sich von der Formel III ableitend) oder einer tautomeren Form davon neu und stellen daher auch einen Gegenstand der vorliegenden Erfindung dar.

Von besonderem Interesse sind Verbindungen der Formel VII, worin $R_1$ unsubstituiertes oder durch ein bis drei gleiche oder verschiedene Substituenten Chlor, Methyl oder Methoxy substituiertes Phenyl bedeutet.

Zu den Verbindungen der Formel VII gelangt man, indem man z.B. ein Aminoderivat der Formel VIII oder einer tautomeren Form davon

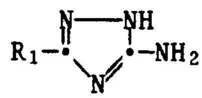

(VIII)

mit einem Malonsäuredialkyl- oder bevorzugt -diphenyl- oder -di-(2,4-dichlorphenyl)ester nach an und für sich bekannten Verfahren umsetzt. Alkyl bedeutet z.B. $C_1$-$C_5$-Alkyl.
Die Verbindungen der Formel VIII sind bekannte Verbindungen und können z.B. durch Umsetzung eines entsprechenden Benzhydrazids der Formel $R_1CONHNH_2$ mit Cyanamid und anschliessenden Ringschluss des erhaltenen Anlagerungsproduktes mittels HCl nach bekannten Verfahren gewonnen werden. In den oben aufgeführten Formeln hat $R_1$ die oben angegebene Bedeutung.

Die nach den oben beschriebenen Methoden hergestellten erfindungsgemässen Isoindoline der Formel I fallen zumeist schon in der Hitze aus und können durch Abfiltrieren und gegebenenfalls durch Waschen mit organischen Lösungsmitteln in reiner Form isoliert werden.

Die erfindungsgemässen Isoindoline der Formel I stellen wertvolle Pigmente dar, welche im allgemeinen eine gute Textur besitzen und meistens als Rohprodukte verwendet werden können. Falls nötig oder erwünscht, kann man die Rohprodukte durch Mahlen oder Kneten in eine feindisperse Form überführen. Dabei werden zweckmässig Mahlhilfsmittel, wie Glas-, Kunststoff-, Stahl- oder Metallmahlkörper, anorganische und/oder organische Salze in Gegenwart oder Abwesenheit organischer Lösungmittel, verwendet. Nach dem Mahlen werden Hilfsmittel wie üblich entfernt, lösliche anorganische Salze z.B. mit Wasser und wasserunlösliche Hilfsmittel beispielsweise durch Wasserdampfdestillation. Auch durch Behandeln der Rohpigmente mit organischen Lösungsmitteln kann oft eine Verbesserung der Pigmenteigenschaften erreicht werden.

Hochmolekulare organische Materialien, die mit den erfindungsgemässen Isoindolinen der Formel I gefärbt bzw. pigmentiert werden können, sind z.B. Celluloseether und -ester, wie Ethylcellulose, Nitrocellulose, Celluloseacetat oder Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, Gummi, Casein, Silikon und Silikonharze, einzeln oder in Mischungen.

Hochmolekulare organische Materialien in gelöster Form als Filmbildner kommen auch in Frage, wie z.B. Leinölfirnis, Nitrocellulose, Alkydharze, Phenolharze, Melaminharze, Acrylharze und Harnstoff-Formaldehydharze.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die Isoindoline der Formel I als Toner oder in Form von Präparaten einzusetzen. Bezogen auf das zu pigmentierende hochmolekulare organische Material kann man die Isoindoline der Formel I z.B. in einer Menge von 0,01 bis 30 Gew.%, vorzugsweise von 0,1 bis 10 Gew.%, einsetzen.

Die Pigmentierung der hochmolekularen organischen Substanzen mit den Isoindolinen der Formel I erfolgt beispielsweise derart, dass man ein solches Isoindolin gegebenenfalls in Form von Masterbatches diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzguss, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Isoindoline in die Polymeren eingearbeitet werden. Es ist ferner möglich, zwecks Erzielung verschiedener Farbtöne den hochmolekularen, organischen Stoffen neben den erfindungsgemässen Isoindolinen noch Füllstoffe bzw. andere farbgebende Bestandteile, wie Weiss-, Bunt- oder Schwarzpigmente, in beliebigen Mengen zuzufügen.

Zum Pigmentieren von Lacken und Druckfarben werden die hochmolekularen organischen Materialien und die erfindungsgemässen Isoindoline gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die

einzelnen Komponenten für sich oder auch mehrere gemeinsam dispergiert bzw. löst, und erst hierauf alle Komponenten zusammenbringt.

Die erhaltenen Ausfärbungen, beispielsweise in Kunststoffen, Fasern, Anstrichstoffen, Lacken oder Drucken, zeichnen sich durch gute allgemeine Eigenschaften, wie z.B. hohe Farbstärke, Transparenz, Farbtonreinheit und Glanz, sowie gute Ueberlackier-, Migrations-, Hitze-, Licht- und Wetterbeständigkeit, aus. Bei optimaler Partikelgrösse können die erfindungsgemässen Isoindoline eine hohe Deckfähigkeit aufweisen.

Die nachfolgenden Beispiele dienen zur Erläuterung der Erfindung.

Beispiel 1:

2,88 g 1-(Cyan-phenylcarbamoylmethylen)-3-imino-isoindolin und 2,74 g der Verbindung der Formel

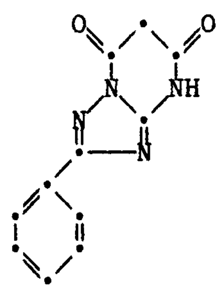

werden in 100 ml Eisessig bei Rückflusstemperatur während 3 Stunden gerührt. Das ausgefallene Produkt wird bei 80 °C filtriert, mit kaltem Eisessig und anschliessend mit kaltem Methanol gewaschen und bei 80-90 °C getrocknet. Man erhält 4,4 g des Isoindolins der Formel

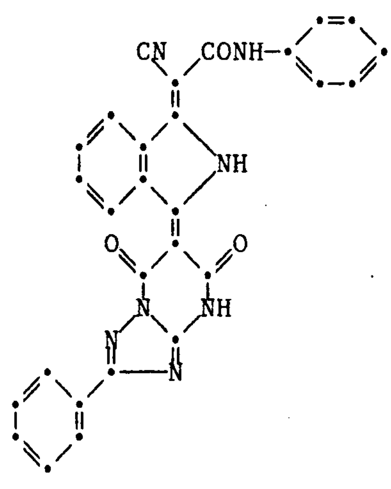

in Form eines orangen Pulvers. Zur Reinigung werden 3 g dieses Produktes in 50 ml Dimethylformamid während 2 Stunden bei 120 °C gerührt. Nach dem Abkühlen und Abfiltrieren der Suspension erhält man 2,3 g eines orangen Pigmentes, das, in Lacke eingearbeitet, farbstarke Ausfärbungen mit hervorragenden Pigmenteigenschaften ergibt.

Beispiel 2:

In analoger Weise wie in Beispiel 1 beschrieben, werden 1:1-Kondensationsprodukte aus 1,3-Diimino-isoindolin und einer Verbindung der Formel $NC-CH_2-R$ mit einer Verbindung der Formel VII, worin $R_1$ Phenyl darstellt, zu Produkten der Formel

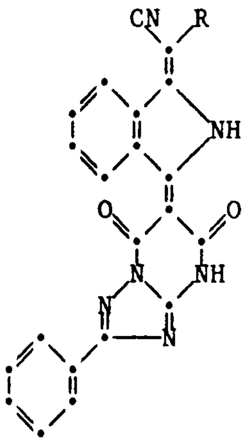

kondensiert, wobei R die in der nachfolgenden Tabelle angeführte Bedeutung hat. Mit diesen Produkten werden nach Einarbeitung in Lacke farbstarke Ausfärbungen mit guten Pigmenteigenschaften erhalten.

Tabelle

| Beispiel Nr. | R | Farbton in einem Alkyd-Melamin-Lack (5 % $TiO_2$, 1 % Pigment) |
|---|---|---|
| 2 | —CONH— 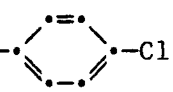 | orange |
| 3 | —CONH— 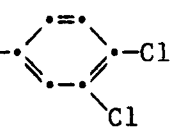 | orange |
| 4 | —CONH— 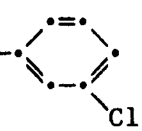 | orange |
| 5 | —CONH— 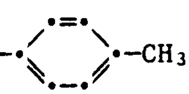 | orange |
| 6 | 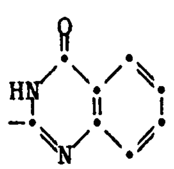 | rot |

Beispiel 7:

24 g 3-Amino-5-phenyl-1,2-4-triazol und 59,1 g Malonsäurebis-2,4-dichlorphenylester werden in 250 ml o-Dichlorbenzol auf 170-180 °C erhitzt. Nach 5 Stunden lässt man die heisse Suspension auf Raumtemperatur abkühlen, und man filtriert den Rückstand ab. Nach dem Waschen mit kaltem Methanol wird das Produkt getrocknet. Man erhält 30,6 g der Verbindung der Formel

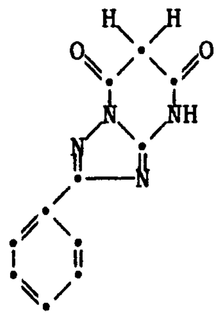

| Mikroanalyse für $C_{11}H_8N_4O_2$: | | | |
|---|---|---|---|
| Ber.: | C 57,9 % | H 3,53 % | N 24,55 % |
| Gef.: | C 57,6 % | H 3,6 % | N 24,6 % |

Schmelzpunkt: über 300 °C.
$\lambda_{max}$ (UV; in nm) in Ethanol: 240, 264, 327 und 341.

Beispiel 8:

Eine Mischung von 130 g Steatitkugeln von 8 mm Durchmesser, 47,5 g eines Alkydmelamineinbrennlackes bestehend aus 60 g kurzöligem Alkydharz ®BECKOSOL 27-320 [60 %ig in Xylol; Reichhold Chemie AG], 36 g des Melaminharzes ®SUPER-BECKAMIN 13-501 [50 %ig in Xylol:Butanol (2:1-Gemisch); Reichhold Chemie AG], 2 g Xylol und 2 g Ethylenglykolmonomethylether, und 2,5 g des nach Beispiel 1 erhaltenen Isoindolins werden in einer 200 ml fassenden Glasflasche mit "Twist-Off"-Verschluss während 120 Stunden auf einem Rollgestell dispergiert. Nach Abtrennen der Steatitkugeln werden 2,4 g der so dispergierten Volltonmischung mit 6 g Titandioxid ®KRONOS RN 59 (KRONOS Titan GmbH) und weiteren 24,0 g des Alkydmelamineinbrennlacks vermischt, das Produkt wird dann auf Aluminiumbleche gespritzt und anschliessend während 30 Minuten bei 130 °C eingebrannt. Man erhält orange Ausfärbungen mit ausgezeichneten Licht- und Wetterbeständigkeiten.

Beispiel 9:

40 mg des gemäss Beispiel 1 erhaltenen Isoindolins werden mit 7,3 ml Dioctylphthalat und 13,3 g eines stabilisierten Polyvinylchlorids vom Typ ®LONZA E 722 in einem Becherglas mit einem Glasstab gut vermischt. Das erhaltene Gemisch wird auf einem Walzenstuhl während 5 Minuten bei 160 °C zu einer dünnen Folie verarbeitet. Die so erzeugte PVC-Folie weist eine opake, migrations-, wetter- und lichtbeständige orange Färbung auf.

## Patentansprüche

1. Isoindoline der Formel I

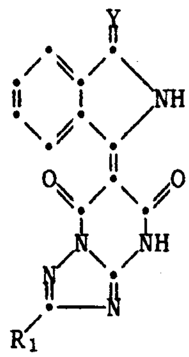

(I),

worin
Y für einen Rest der Formeln II, III, IV, IVa, V oder VI

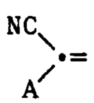

,

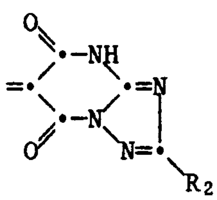

,

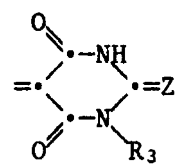

,

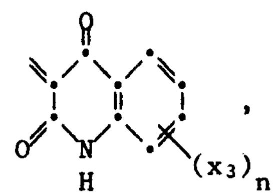

,

(II) (III) (IV) (IVa)

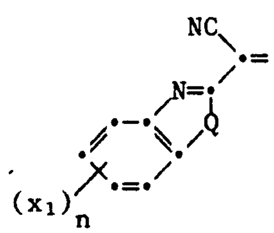

,

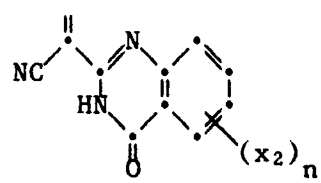

(V) (VI)

steht, wobei

$R_1$ und $R_2$ unabhängig voneinander -H, $-CONH_2$, $-COOC_1$-$C_4$-Alkyl, $C_1$-$C_3$-Alkyl, unsubstituiertes oder durch ein bis drei gleiche oder verschiedene Substituenten bestehend aus den Gruppen Halogen, $C_1$-$C_3$-Alkyl oder $C_1$-$C_3$-Alkoxy substituiertes Phenyl bedeuten, $R_3$ -H, $C_1$-$C_4$-Alkyl oder unsubstituiertes oder durch ein bis zwei gleiche oder verschiedene Substituenten bestehend aus den Gruppen Halogen, $C_1$-$C_3$-Alkyl oder $C_1$-$C_3$-Alkoxy substituiertes Phenyl ist, Z und Q für O, S oder NH stehen, und A eine Gruppe der Formeln -CN, -COO-$C_1$-$C_4$-Alkyl oder $-CONHR_4$ ist, wobei $R_4$ -H, $C_1$-$C_4$-Alkyl, Benzyl, unsubstituiertes oder ein- oder mehrfach gleich oder verschieden durch Halogen, $C_1$-$C_4$-Alkyl, $C_1$-$C_4$-Alkoxy, eine Gruppe $-COR_5$, $-NHCOC_1$-$C_4$-Alkyl und

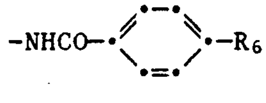

substituiertes Phenyl ist, worin $R_5$ $C_1$-$C_4$-Alkoxy, $-NH_2$ oder $-NHC_1$-$C_4$-Alkyl, und $R_6$ Wasserstoff, Halogen oder $C_1$-$C_4$-Alkyl bedeuten, ferner $R_4$ unsubstituiertes oder durch Halogen, Methyl oder Methoxy mono- oder disubstituiertes Naphthyl ist, und $x_1$, $x_2$ und $x_3$ unabhängig voneinander Halogen,

Methyl, Methoxy oder $-NHCOCH_3$ und n die Zahl 0, 1 oder 2 darstellen.

**2.** Isoindoline der Formel I gemäss Anspruch 1, worin Y für einen Rest der Formel

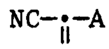

steht, und $R_1$ unsubstituiertes oder durch ein bis drei gleiche oder verschiedene Substituenten bestehend aus den Gruppen Chlor, Methyl oder Methoxy substituiertes Phenyl bedeutet, und A eine Gruppe der Formel $-CONHR_4$ ist, wobei $R_4$ unsubstituiertes oder ein-oder zweifach gleich oder verschieden durch -Cl, $-CH_3$, $-OCH_3$ $-COOCH_3$, $-COOC_2H_5$, $-CONH_2$, $-NHCOCH_3$ und $-NHCOC_6H_5$ substituiertes Phenyl bedeutet.

**3.** Isoindoline der Formel I gemäss Anspruch 1, worin $R_1$ Phenyl und Y eine Gruppe der Formel

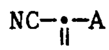

bedeuten, wobei A unsubstituiertes oder durch 1 oder 2 Chloratome substituiertes Phenyl ist.

**4.** Verbindungen der Formel VII

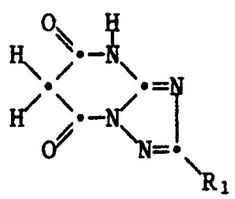

(VII),

worin $R_1$ -H, $-CONH_2$, $-COOC_1$-$C_4$-Alkyl, $C_1$-$C_3$-Alkyl, unsubstituiertes oder durch ein bis drei gleiche oder verschiedene Substituenten bestehend aus den Gruppen Halogen, $C_1$-$C_3$-Alkyl oder $C_1$-$C_3$-Alkoxy substituiertes Phenyl bedeutet.

**5.** Verbindungen der Formel VII gemäss Anspruch 4, wobei $R_1$ unsubstituiertes oder durch ein bis drei gleiche oder verschiedene Substituenten Chlor, Methyl oder Methoxy substituiertes Phenyl bedeutet.

**6.** Verfahren zum Färben von hochmolekularem organischem Material, dadurch gekennzeichnet, dass ein Isoindolin der Formel I gemäss Anspruch 1 verwendet wird.

**7.** Gemäss Patentanspruch 6 gefärbtes hochmolekulares organisches Material.

**Claims**

**1.** An isoindoline of formula I

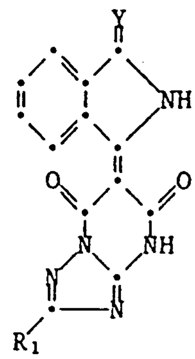

(I)

in which
Y is a radical of formula II, III, IV, IVa, V or VI

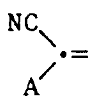

,

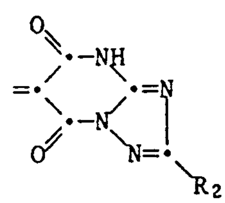

,

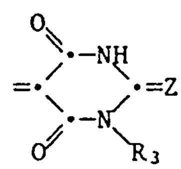

,

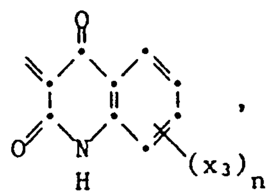

,

(II) (III) (IV) (IVa)

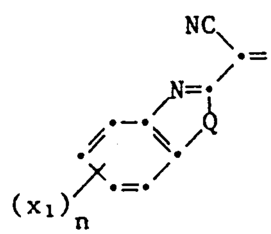

,

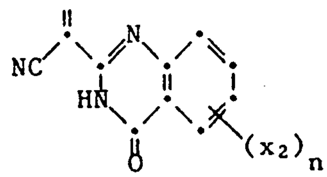

(V) (VI)

in which $R_1$ and $R_2$ are each independently of the other -H, $-CONH_2$, -COO-$C_1$-$C_4$alkyl, $C_1$-$C_3$alkyl, phenyl which is unsubstituted or substituted by one to three identical or different substituents selected from the group consisting of halogen, $C_1$-$C_3$alkyl and $C_1$-$C_3$alkoxy, $R_3$ is -H, $C_1$-$C_4$alkyl, phenyl which is unsubstituted or substituted by one or two identical or different substituents selected from the group consisting of halogen, $C_1$-$C_3$alkyl and $C_1$-$C_3$alkoxy, Z and Q are O, S or NH, and A is a group of formula -CN, -COO-$C_1$-$C_4$alkyl or $-CONHR_4$, in which $R_4$ is -H, $C_1$-$C_4$alkyl, benzyl, phenyl which is unsubstituted or substituted by one or more identical or different members selected from the group consisting of halogen, $C_1$-$C_4$alkyl, $C_1$-$C_4$alkoxy, a group $-COR_5$, -NHCO-$C_1$-$C_4$alkyl and

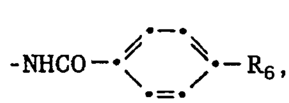

in which $R_5$ is $C_1$-$C_4$alkoxy, $-NH_2$ or -NH-$C_1$-$C_4$alkyl, and $R_6$ is hydrogen, halogen or $C_1$-$C_4$alkyl, and $R_4$ is also naphthyl which is unsubstituted or monosubstituted or disubstituted by halogen, methyl or

methoxy, and $x_1$, $x_2$ and $x_3$ are each independently of one another halogen, methyl, methoxy or $-NHCOCH_3$, and n is 0, 1 or 2.

**2.** An isoindoline of formula I according to claim 1, in which Y is a radical of formula

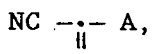

and $R_1$ is phenyl which is unsubstituted or substituted by one to three identical or different substituents selected from the group consisting of chlorine, methyl and methoxy, and A is a group of formula $-CONHR_4$, in which $R_4$ is phenyl which is unsubstituted or substituted by one or two identical or different members selected from the group consisting of -Cl, $-CH_3$, $-OCH_3$, $-COOCH_3$, $-COOC_2H_5$, $-CONH_2$, $-NHCOCH_3$ and $-NHCOC_6H_5$.

**3.** An isoindoline of formula I according to claim 1, in which $R_1$ is phenyl and Y is a group of formula

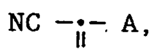

in which A is phenyl which is unsubstituted or substituted by 1 or 2 chlorine atoms.

**4.** A compound of formula VII

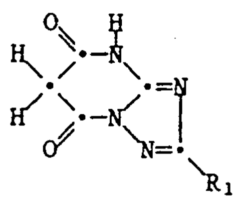

(VII)

in which $R_1$ is -H, $-CONH_2$, -COO-$C_1$-$C_4$alkyl, $C_1$-$C_3$alkyl, phenyl which is unsubstituted or substituted by one to three identical or different substituents selected from the group consisting of halogen, $C_1$-$C_3$alkyl and $C_1$-$C_3$alkoxy.

**5.** A compound of formula VII according to claim 4, in which $R_1$ is phenyl which is unsubstituted or substituted by one to three identical or different substituents selected from the group consisting of chlorine, methyl and methoxy.

**6.** A process for colouring organic material of high molecular weight, which comprises the use of an isoindoline of formula I according to claim 1.

**7.** An organic material of high molecular weight coloured according to patent claim 6.

## Revendications

**1.** Isoindolines de formule I :

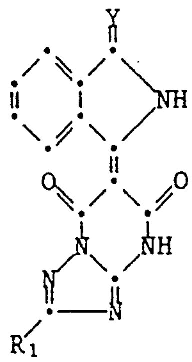

(I)

dans laquelle
Y est un radical ayant les formules II, III, IV, IVa, V ou VI :

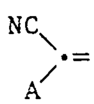

,

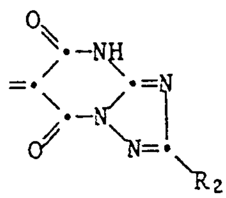

,

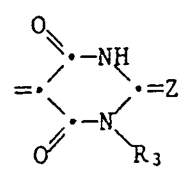

,

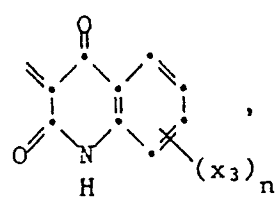

,

(II) (III) (IV) (IVa)

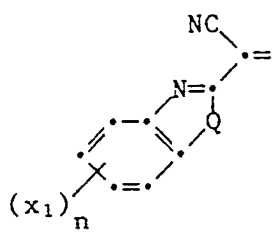

,

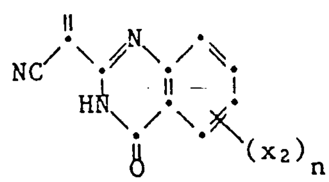

(V) (VI)

où $R_1$ et $R_2$, indépendamment l'un de l'autre, sont chacun -H ou un radical $-CONH_2$, -COO(alkyle en $C_1$-$C_4$), alkyle en $C_1$-$C_3$, phényle non substitué, ou substitué par un à trois substituants identiques ou différents constitués des groupes halogéno, alkyle en $C_1$-$C_3$ ou alcoxy en $C_1$-$C_3$, $R_3$ est -H ou un radical alkyle en $C_1$-$C_4$ ou un radical phényle non substitué, ou substitué par un ou deux substituants identiques ou différents constitués des groupes halogéno, alkyle en $C_1$-$C_3$ ou alcoxy en $C_1$-$C_3$, Z et Q sont chacun O, S ou NH, et A est un groupe de formules -CN, -COO-(alkyle en $C_1$-$C_4$) ou $-CONHR_4$, où $R_4$ est -H ou un radical alkyle en $C_1$-$C_4$, benzyle, phényle non substitué, ou substitué une ou plusieurs fois par des substituants identiques ou différents constitués des groupes halogéno, alkyle en $C_1$-$C_4$, alcoxy en $C_1$-$C_4$, $-COR_5$, -NHCO(alkyle en $C_1$-$C_4$) ou

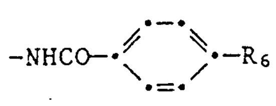 :

où $R_5$ est un radical alcoxy en $C_1$-$C_4$, $-NH_2$ ou -NH(alkyle en $C_1$-$C_4$) et $R_6$ est un hydrogène, un halogène ou un radical alkyle en $C_1$-$C_4$, $R_4$ représentant de plus un radical naphtyle non substitué ou

mono- ou disubstitué par des radicaux halogéno, méthyle ou méthoxy, et $x_1$, $x_2$ et $x_3$ indépendamment les uns des autres, représentent chacun un radical halogéno, méthyle, méthoxy ou $-NHCOCH_3$, et n vaut 0, 1 ou 2.

**2.** Isoindolines de formule I, dans laquelle Y est un résidu de formule

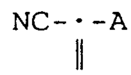

et $R_1$ est un radical phényle non substitué ou substitué par un à trois substituants identiques ou différents choisis parmi l'ensemble comprenant les radicaux chloro, méthyle ou méthoxy, et A est un groupe de formule $-CONHR_4$ où $R_4$ est un radical phényle non substitué ou une ou deux fois substitué par des substituants identiques ou différents choisis parmi l'ensemble comprenant -Cl, $-CH_3$, $-OCH_3$, $-COOCH_3$, $-COOC_2H_5$, $-CONH_2$, $-NHCOCH_3$ et $-NHCOC_6H_5$.

**3.** Isoindolines de formule I dans laquelle $R_1$ est un radical phényle et Y est un groupe de formule

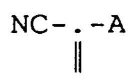

où A est un radical phényle non substitué ou substitué par un ou deux atomes de chlore.

**4.** Composés de formule VII :

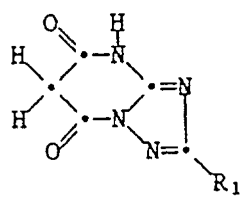

(VII)

dans laquelle $R_1$ est -H ou un radical $-CONH_2$, -COO(alkyle en $C_1$-$C_4$), alkyle en $C_1$-$C_3$ ou phényle non substitué, ou substitué par un à trois substituants identiques ou différents constitués des groupes halogéno, alkyle en $C_1$-$C_3$ ou alcoxy en $C_1$-$C_3$.

**5.** Composés de formule VII selon la revendication 4, dans lesquels $R_1$ est un radical phényle non substitué, ou substitué par un à trois substituants identiques ou différents choisis parmi l'ensemble comprenant les radicaux chloro, méthyle ou méthoxy.

**6.** Procédé pour colorer un matériau organique à masse moléculaire élevée, caractérisé en ce qu'on utilise une isoindoline de formule I selon la revendication 1.

**7.** Matériau organique à masse moléculaire élevée, coloré selon la revendication 6.